# EUROPÄISCHE PATENTANMELDUNG

(11) **EP 4 046 594 A1**
(43) Veröffentlichungstag der Anmeldung: **24.08.2022**
(21) Anmeldenummer: 21158015.4
(22) Anmeldetag: 18.02.2021
(51) Int. Cl.: A61C 19/10, A61C 19/04

(54) **VERFAHREN ZUR FESTLEGUNG EINER ZAHNFARBE**

(71) Anmelder: Ivoclar Vivadent AG, 9494 Schaan (LI)
(72) Erfinder: Die Erfindernennung liegt noch nicht vor
(74) Vertreter: Baronetzky, Klaus

(57) **Zusammenfassung**

Es ist ein Verfahren zur Festlegung einer Zahnfarbe vorgesehen, wobei eine Auswertvorrichtung einen iterativen lernenden Algorithmus aufweist, der, in einem vorbereitenden Schritt, basierend auf mindestens einer vorbekannten, ggf. virtuell im RGB-Raum erzeugten, Musterzahnfarbe, Bilder bei unterschiedlichen Lichtverhältnissen und Aufnahmewinkeln erfasst und auswertet und die Zuordnung zu der zutreffenden Musterzahnfarbe lernt. In einem Auswertschritt ist eine Aufnahmevorrichtung vorgesehen, mit welcher ein Bild eines Hilfskörpers mit vorbekannter Musterzahnfarbe zusammen mit mindestens einem Zahn aufgenommen wird. Die Aufnahmevorrichtung erfasst mindestens zwei Bilder der Kombination des zu bestimmenden Zahns und des Hilfskörpers aus unterschiedlichen Aufnahmewinkeln und leiter diese der Auswertvorrichtung zu. Die Auswertvorrichtung wertet, basierend auf der gelernten Zuordnung zu der zutreffenden Musterzahnfarbe, die erfassten Bilder aus und gibt die Zahnfarbe des zu bestimmenden Zahns gemäß einem Referenzwert, wie einem gängigen Zahnschlüssel, z.B. A1, B2 usw., aus.

## Beschreibung

Die Erfindung betrifft ein Verfahren zur Festlegung einer Zahnfarbe, gemäß dem Oberbegriff von Anspruch 1.

Aus der EP 3 613 382 A1 ist es bekannt geworden, einen Farbselektionskörper zu verwenden, der neben einen Zahn, dessen Zahnfarbe zu bestimmen ist, gehalten wird. Es wird ein gemeinsames Bild des Zahns und des als Hilfskörper ausgebildeten Farbselektionskörper aufgenommen. Da der Hilfskörper eine bekannte Zahnfarbe aufweist, lässt sich hierdurch leichter und genauer die Farbe des Zahns ermitteln.

Die vorstehend genannte Lösung erfordert nach Möglichkeit reproduzierbare Lichtverhältnisse. Zwar lässt sich durch das Vorhandensein des Hilfskörpers mit der bekannten Zahnfarbe die erfasste Farbe des Zahns kalibrieren bzw. normieren. Es hat sich jedoch gezeigt, dass trotz dieser Möglichkeit in der Praxis Abweichungen entstehen, sodass die im Labor realisierbare Genauigkeit der Farbermittlung in der Praxis nicht besteht.

Demgegenüber liegt der Erfindung die Aufgabe zugrunde, ein Verfahren zur Festlegung einer Zahnfarbe gemäß dem Oberbegriff von Anspruch 1 zu schaffen, dass genaue Farbermittlungen auch in der Praxis sicherstellt.

Diese Aufgabe wird erfindungsgemäß durch Anspruch 1 gelöst. Vorteilhafte Weiterbildungen ergeben sich aus den Unteransprüchen.

Erfindungsgemäß ist es vorgesehen, eine Auswertvorrichtung zunächst in einem iterativen lernenden Algorithmus Bilder von Musterzähnen bei unterschiedlichen Lichtverhältnissen und Aufnahmewinkeln erfasst und auswertet und eine Zuordnung der je erfassten Bilder zu der bekannten zutreffenden Musterzahnfarbe lernt.

Insbesondere die Erfassung und das Lernen bei unterschiedlichen Aufnahmewinkeln ist erfindungsgemäß wichtig und trägt signifikant zur Verbesserung der Erkennungsfähigkeit der Auswertvorrichtung bei.

Für die Aufnahme der Bilder von Musterzähnen wird eine Erstaufnahmevorrichtung eingesetzt. Diese Erstaufnahmevorrichtung kann von besonders hoher Qualität sein. Beispielsweise kann eine professionelle Spiegelreflexkamera eingesetzt werden um, deren Rohdaten ausgelesen werden können. Die Qualität der Rohdaten ist typischerweise besser als die der in ein Standardformat wie JPG konvertierten Daten der Kamera.

Es ist aber auch möglich, als Erstaufnahmevorrichtung eine Kamera eines Endgeräts einzusetzen, beispielsweise diejenige eines Smartphones.

Eine Aufnahmevorrichtung, z.B. eine weitere Aufnahmevorrichtung, wird in einem Auswertschritt eingesetzt. In dem Auswertschritt wird ein Bild eines Zahnes, dessen Farbe bestimmt werden soll, zusammen mit dem Hilfskörper mit der bekannten Farbe aufgenommen.

Die Auswertvorrichtung greift auf die gelernten Bilder und die mit diesen erfassten Bilddaten zu.

Die Auswerteinrichtung weist bevorzugt einen ersten Teil auf, der der in dem vorbereitenden Schritt zum Einsatz kommt, und einen zweite Teil, der in der Praxis dann der Auswertvorrichtung zum Einsatz kommt.

Der zweite Teil greift auf die gleichen Daten wie der erste Teil zu.

Damit lässt sich bewerkstelligen, dass die Auswertvorrichtung vor Ort, also beispielsweise in einer Zahnarztpraxis, eingesetzt werden kann, ohne dass auf die vorbereitend gewonnenen Daten verzichtet werden müsste.

Um einen leichten Zugriff auf die gewonnenen Daten und Erkenntnisse zu ermöglichen, ist es bevorzugt, dass diese in einer Cloud oder jedenfalls in einem Bereich abgespeichert sind, der zum einen geschützt ist und zum anderen in der Praxis zugänglich ist.

In der Praxis kommt dann der zweite Teil der Auswertvorrichtung zum Einsatz, der auf die gleichen Daten wie der erste Teil zugreift.

Diese Daten stehen also der Auswertvorrichtung stets zur Verfügung. Dies bedeutet allerdings nicht, dass bei dem erfindungsgemäßen Verfahren allzeit ein Vollzugriff auf die Daten erforderlich ist.

Bevorzugt ist es vielmehr, dass die Auswertvorrichtung im zweiten Teil einen Speicher aufweist, dessen Inhalt periodisch mit der Cloud synchronisiert wird.

Was der zweite Teil der Auswertvorrichtungen dann macht, ist eine Auswertung der von der - weiteren - Aufnahmevorrichtung erfassten Bilder und eine Zuordnung dieser zu Musterzahnfarben, basierend auf der gelernten Zuordnung zu Musterzahnfarben, und dann die Ausgabe der Zahnfarbe nach einem gängigen Zahnschlüssel basierend wiederum darauf.

Hierbei muss die Musterzahnfarbe nicht real vorliegen und abgespeichert sein; eine virtuelle Erzeugung der Musterzahnfarbe, also die Definition in einem festgelegten Farbraum ist vielmehr ausreichend. Es ist auch möglich, eine Zahnfarbe lediglich numerisch in einem virtuellen Farbraum, bevorzugt im RGB-Raum, zu erzeugen, und diese als Referenz zu verwenden. Eine solche Farbe wir hier als RGB-Zahnfarbe bezeichnet, wobei es sich versteht, dass hierdurch auch in anderen virtuellen Zahnräumen erzeugte Farben mit umfasst sein sollen.

Es gibt mehrere Möglichkeiten, eine solche virtuelle Zahnfarbe zu erzeugen:
1. Man verwendet einen Scan eines Zahnes und ermittelt die zugehörigen RGB-Werte;
2. Man verwendet die Werte bestehender Zahnbibliotheken; oder
3. Man verwendet Farbmessgeräte wie Fotospektrometer, um die Farben numerisch festzulegen.

Überraschend ergibt sich durch die Vielzahl von Aufnahmesituationen in dem vorbereitenden Schritt eine deutlich bessere Erkennung und Festlegung der tatsächlichen Farbe des zu bestimmenden Zahns.

Zu den Aufnahmesituationen gehören solche, bei denen mit unterschiedlichen Lichtquellen und Helligkeiten gearbeitet wird.

Beispielsweise können die gleichen Musterzähne mit dem Lichtspektrum einer Halogenlampe, einer LED-Lampe und von Tageslicht bei Sonnenschein und zum anderen bei bewölktem Himmel aufgenommen werden. Dies zudem bei drei verschiedenen Helligkeitsstufen. Und zudem bei 5 bis 15 in vertikaler und in horizontaler Richtung verschiedenen Aufnahmewinkeln.

Diese Vorbereitungen führt zu einer profunden Datenbasis von z.b. 100 bis 300 verschiedenen Aufnahmesituationen für jeden Musterzahn.

Es versteht sich, dass die vorstehenden die Erläuterung lediglich beispielhaft ist und insbesondere die Erfindung nicht auf die Anzahl von Aufnahmesituationen in dem vorbereitenden Schritt beschränkt ist.

In vorteilhafter Ausgestaltung des erfindungsgemäßen Verfahrens ist es vorgesehen, in dem vorbereitenden Schritt bei der Iteration zu prüfen, inwiefern sich in jeder Iteration das Ergebnis ändert. Wenn die Ergebnisänderungen geringer als ein vorgegebener Schwellwert ist, wird davon ausgegangen, dass die erwünschte Genauigkeit erreicht ist.

Diese Ausgestaltung kann auch dahingehend modifiziert werden, dass erst nach mehrmaligem Unterschreiten des Schwellwerts der Änderung die Iteration abgebrochen wird.

Bevorzugt wird nach der Beendigung des vorbereitenden Schritt dafür gesorgt, dass sämtliche ermittelten Daten, die die Zuordnung zwischen dem Ergebnis des vorbereitenden Schritt und den Musterzahnfarben umfassen, in eine Cloud verbracht werden, in welcher sie im Zugriff sind, wenn dies erforderlich ist.

Bevor von einem Endgerät ein Auswertschritt vorgenommen wird, nimmt es einen Datenabgleich vor, dahingehend, dass die ermittelten Daten soweit erforderlich und insbesondere vollständig lokal auf dem Endgerät gespeichert werden.

Diese Daten werden regelmäßig synchronisiert, so dass Änderungen regelmäßig auf die Endgeräte gelangen.

Wenn das Endgerät den Auswertschritt ausführen soll, stehen also stets die aktuellen Daten der Auswertvorrichtung, soweit sie im vorbereitenden Schritt erbracht und bereitgestellt wurden, zur Verfügung.

Der erste Teil der Auswertvorrichtung ist ausschließlich im vorbereitenden Schritt tätig, steht aber weiter zur Verfügung, wenn weitere Lichtverhältnisse oder Aufnahmewinkel. erfasst werden sollen.

In dem Fall, dass die in der Praxis relevanten Lichtverhältnisse und Aufnahmewinkel zwar erfasst sind, aber ggf. noch Detailanpassungen sinnvoll sind, kann die Auswertvorrichtung auch als ausführbares oder compilerbares Programm in die Cloud verbracht werden.

Diese Lösung hat den Vorteil, dass ein Zahnarzt bei Bedarf auch die Auswertvorrichtung in ihrem ersten Teil selbst nutzen kann, wenn er über die erforderliche Ausrüstung verfügt, und selbst seine besonderen Lichtverhältnisse berücksichtigen kann, unter der Voraussetzung, dass ihm Musterzahnfarben zur Verfügung stehen, die er für die Durchführung des ersten Schritt benötigt.

Die von ihm so ermittelten und hinsichtlich der besonderen Lichtverhältnisse neuen Daten kann der Zahnarzt dann gewünschtenfalls in der Cloud anderen Zahnärzten zur Verfügung stellen.

Eine vergleichbare Handhabung ist auch möglich, wenn eine regionale Anpassung erwünscht ist:
Das Lichtspektrum von Tageslicht ist geographisch unterschiedlich zwischen äquatornahen und polnahen Gegenden, da in polnahen Gegenden die Absorptionsbande der Lufthülle deutlich stärker zum Tragen kommen.

Wenn in dem ersten Schritt ein Mittelwert des Tageslichts zugrundegelegt wird, ist es möglich, dass ein Zahnarzt in einer äquatornahen Gegenden basierend auf den von ihm durch die Auswertvorrichtung gelieferten Daten zur Auffassung gelangt, dass die eingestellten Tageslichtdaten einer Korrektur bedürfen.

Die von ihm regional angepassten Daten kann er dann beispielsweise in der Cloud anderen Zahnärzten in seiner Gegend zur Verfügung stellen.

Dies ist nur ein Beispiel der erfindungsgemäß bevorzugten Möglichkeiten, einen Datentausch der bereitgestellten Daten in der Cloud zu ermöglichen. Es versteht sich, dass es auch ein Datentausch aus anderen Gründen erfindungsgemäß umfasst ist.

Für die eigentliche Auswertung in der Auswertvorrichtung ist es günstig, wenn auf dem Hilfskörper Referenzpunkte angebracht sind. Die Referenzpunkte sind so gewählt, dass sie von der Auswertvorrichtung erkannt werden können. Wenn beispielsweise drei oder vier Referenzpunkte vorgesehen sind, lässt sich aus deren Anordnung im aufgenommenen Bild rückschließen, in welchem Winkel die Aufnahme vorgenommen wurde.

Erfindungsgemäß günstig ist es dann, wenn aus dieser Winkelerfassung ein Rückschluss bzw. ein Abgleich mit den Daten erfolgt, die dem betreffenden Aufnahmewinkel zugeordnet sind.

In einer weiteren vorteilhaften Ausgestaltung ist eine Aufteilung des aufgenommenen Bildes in Segmente vorgesehen.

Die Segmentierung hat den Vorteil, dass Bereiche ausgeblendet werden können, deren in dem Auswertschritt erfasste Daten darauf schließen lassen, dass Reflexionen vorliegen.

Überraschend lässt sich durch diese Maßnahme die Präzision der Auswertungen deutlich steigern, gerade auch im hellen und damit reflektionsträchtigen Umgebungen.

Für die durch die Auswertvorrichtung vorgenommene Auswertung lassen sich verschiedene Parameter einsetzen:
Beispielsweise ist es möglich, mittels eines Umgebungslichtsensors eine Normierung auf die Helligkeit der Umgebung vorzunehmen. Typischerweise verfügten Endgeräte wie Smartphones über einen Umgebungslichtsensor, der typischerweise die Helligkeit des Displays anpasst.

In vorteilhafter Ausgestaltung der Erfindung wird dieser Umgebungslichtsensor ausgenutzt, um die vorstehend erwähnte Normierung vorzunehmen.

Hochwertige Smartphones können auch spektral zwischen Kunstlicht und Tageslicht unterscheiden. Diese Unterscheidung erfolgt ebenfalls über den eingebauten Umgebungslichtsensor. Auch dieses Unterscheidungsergebnis lässt sich erfindungsgemäß bevorzugt ausnutzen, indem von vornherein die nicht zutreffenden Daten der Auswertvorrichtung nicht zur Verfügung gestellt werden.

Weitere Vorteile, Einzelheiten und Merkmale ergeben sich aus der nachfolgenden Beschreibung eines Ausführungsbeispiels des erfindungsgemäßen Verfahrens anhand der Zeichnung.

Es zeigen:
- Fig. 1: ein schematisches Flussdiagramm des erfindungsgemäßen vorbereitenden Schritts;
- Fig. 2: ein schematisches Flussdiagramm der Bild-Pipeline als Unterprogramm, das sowohl in dem vorbereitenden Schritt als auch in dem Auswertschritt zum Einsatz gelangt; und
- Fig. 3: ein schematisches Flussdiagramm des Auswertschritts.

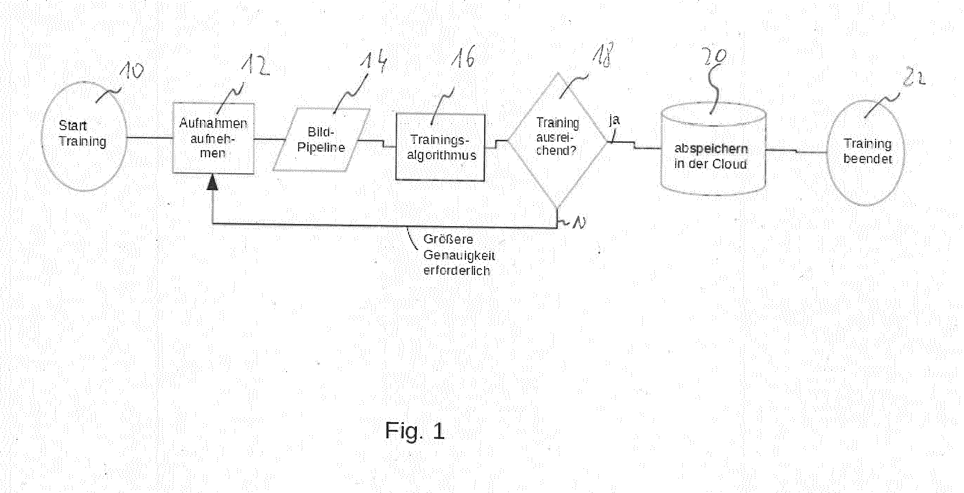

In Fig. 1 ist der vorbereitende Schritt 12 des erfindungsgemäßen Verfahrens zur Festlegung einer Zahnfarbe schematisch dargestellt. Der vorbereitende Schritt 12 wir hier als Training 10 bezeichnet und beginnt gemäß dem Flussdiagramm von Fig. 1 mit dem Start des Trainings 10.

Zunächst werden Bilder, die auch als "Aufnahmen" bezeichnet werden können, aufgenommen, im Schritt 12. Die Aufnahmen stellen Musterzahnfarben dar und die entsprechenden, als Muster ausgebildeten Zähne werden bei unterschiedlichen Lichtverhältnissen und Aufnahmewinkeln aufgenommen, erfasst und ausgewertet.

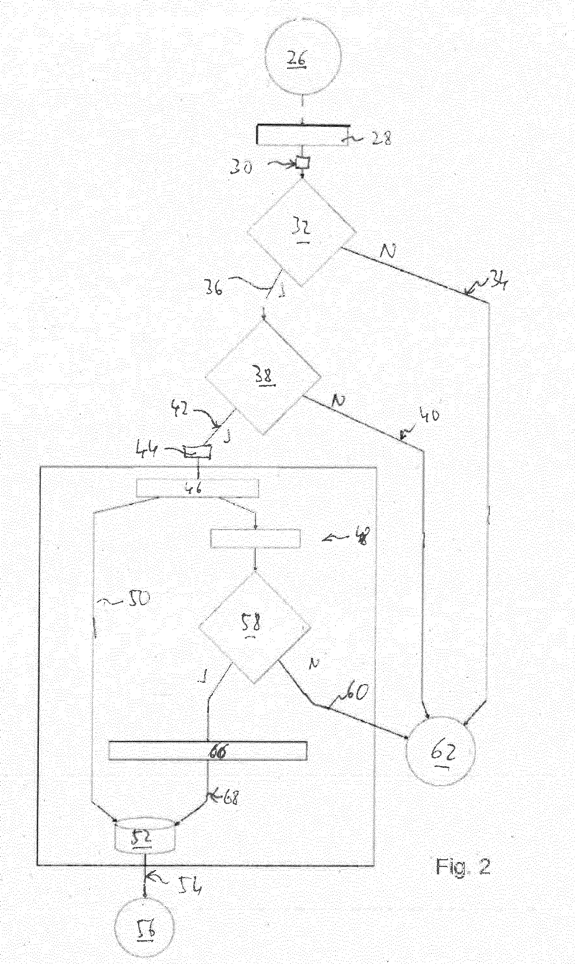

Die aufgenommenen Daten werden einer Bild-Pipeline 14 als einem Unterprogramm zugeleitet, deren Ausgestaltung aus Fig. 2 ersichtlich ist.

Nach der Verarbeitung in der Bild-Pipeline 14 werden die aufbereitenden Daten einem Trainingsalgorithmus 16 zugeleitet. Dieser führt das eigentliche Training durch, also die optimierte Wiedergabe der Musterzahnfarben bei den unterschiedlichen Lichtverhältnissen und Aufnahmewinkeln von Form von Daten.

Anschließend hieran wird im Schritt 18 geprüft, ob das Training ausreichend war. Wenn dies nicht der Fall gewesen ist, also eine größere Genauigkeit erforderlich ist, wird zum Block 12 zurückgesprungen und die erfassten Daten durchlaufen erneut die Bild-Pipeline 14.

Wenn hingegen das Training als ausreichend angesehen wird, werden die "trainierten" Daten in der Cloud abgespeichert, im Schritt 20. Damit ist das Training beim Block 22 beendet.

Aus Fig. 2 sind die einzelnen Schritte der Bild-Pipeline 14 ersichtlich. Die Bild-Pipeline 14 wir im Schritt 26 gestartet. Es liegen Bilddaten 28 vor, die im Schritt 28 in dem Bildspeicher abgespeichert werden.

Das Bild 30 liegt nun vor und es wird hinsichtlich seiner Auflösung und seines Formats im Schritt 32 geprüft. Wenn die Auflösung und das Format nicht ausreichend sind, wird der Pfad 34 eingeschlagen, während dann, wenn sowohl Format als auch Auflösung ausreichend sind, die Ausführung mit dem Pfad 36 weitergeführt wird.

Das Bild 30 wird im Pfad 36 daraufhin geprüft, im Schritt 38, wo die Referenzpunkte des Referenzobjektes sind; diese werden erfasst. Wiederum ergibt sich die Möglichkeit, im Pfad 40, dass keine oder nicht ausreichende Referenzpunkte ermittelt werden konnten.

Wenn hingegen Referenzpunkte gefunden werden konnten, wird der Pfad 42 weiter ausgeführt und es wird im Block 44 die relevante Information aus dem Bild 30 extrahiert. Hierzu gehören gemäß dem Block 46 Farbwerte, die aus dem Referenzobjekt extrahiert werden.

Es wird geprüft, ob ein Zahnsegment im Bild 30 besteht. Wenn dies der Fall ist, wird der Pfad 48 eingeschlagen.

Parallel hierzu werden mit dem Pfad 50 Farbwerte verarbeitet. Die Farbwerte werden bei Block 52 dem Algorithmus zugeleitet, der daraus im Pfad 54 die Farbinformation des Bildes 30 erzeugt, und im Block 56 erfolgt die Datenübergabe an das aufrufende Programm, also das Ende des Unterprogramm Bild-Pipeline.

Gemäß dem Pfad 48 werden auch die Zahnsegmentdaten weiter verarbeitet. Im Block 58 wird geprüft, ob Reflektionen vorliegen. Wenn diese oberhalb eines Schwellwerts liegen, wird der Pfad 60 eingeschlagen, der ebenso wie der Pfad 34 und der Pfad 40 damit endet, dass gemäß dem Block 62 kein Ergebnis vorliegt.

Wenn hingegen gemäß dem Pfad 64 die Reflektionen unterhalb eines Stellwertes liegen, werden dominante Zahnfarben durch das sogenannte k-means-clustering berechnet. Dies geschieht in Block 66.

Hieraus ergeben sich Farbwerte im Pfad 68, die wiederum dem Algorithmus 52 zugeleitet werden.

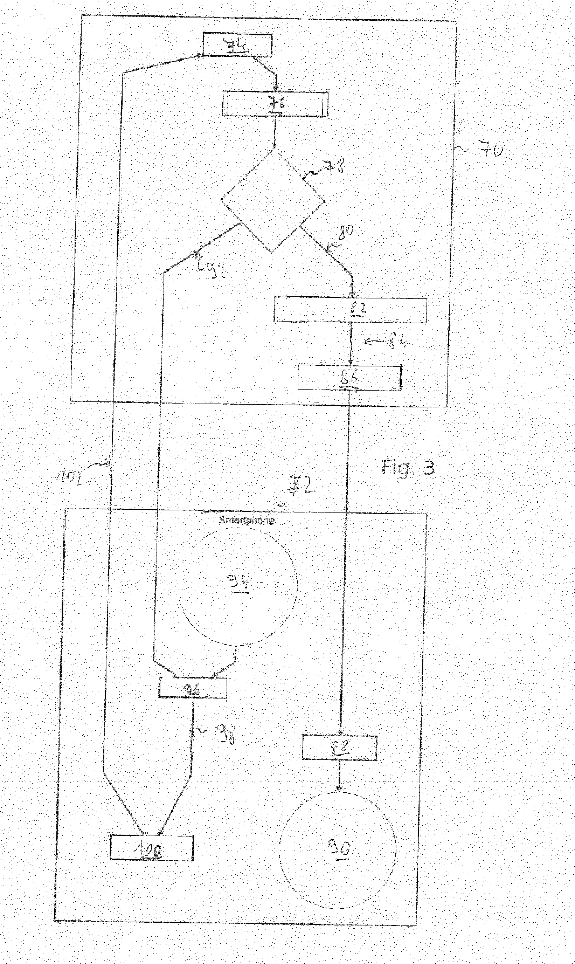

Aus Fig. 3 ist der Auswertschritt ersichtlich. Dieser ist dafür bestimmt, im Endgerät ausgeführt zu werden, beispielsweise einem Smartphone. Ein erster Block 70 stellt die Cloud dar, und ein zweiter Block 72 das Smartphone.

In vorteilhafter Ausgestaltung werden die Daten des vom Endbenutzer erfassten Bildes im Block 74 der Cloud zugeleitet, und es wird im Block 76 in die Bild-Pipeline eingetreten (Fig. 2). Diese Bild-Pipeline ist die gleiche wie die Bild-Pipeline 14 in Fig. 1.

Nach Aufbereitung und Auswertung der Daten wird im Schritt 78 geprüft, ob genug Bilder vorliegen. Wenn dies der Fall ist, wird der Pfad 80 eingeschlagen, und es wird im Block 82 ein Klassifikationsalgorithmus durchgeführt. Ausgangsseitig des Blocks 82 liegen damit bei Block 84 klassifizierte Farben vor. Diese werden über den Block 86 zum Smartphone 72 geleitet.

Das Smartphone empfängt im Block 88 die Daten und im Block 90 ist die Farbklassifikation beendet.

Wenn hingegen im Schritt 78 festgestellt wird, dass nicht genug Bilder vorliegen, wird der Pfad 92 eingeschlagen. In diesem Fall wird die Farbklassifikation im Block 94 vom Smartphone gestartet, und es wird bei 96 eine Aufnahme aufgenommen.

Das Aufnehmen der Aufnahme oder des Bildes wird damit über den Pfad 92 getriggert oder ausgelöst.

Ausgangsseitig des Smartphone 72 liegt ein Bild im Pfad 98 vor. Im Block 100 wird dieses Bild über den Pfad 102 zur Cloud geleitet und dort geladen, so dass die Ausführung im Block 74 bei diesem Bild starten kann.

## Patentansprüche

1. Verfahren zur Festlegung einer Zahnfarbe, wobei
- eine Auswertvorrichtung einen iterativen lernenden Algorithmus aufweist, der, in einem vorbereitenden Schritt, basierend auf mindestens einer vorbekannten, ggf. virtuell im RGB-Raum erzeugten, Musterzahnfarbe, Bilder bei unterschiedlichen Lichtverhältnissen und Aufnahmewinkeln erfasst und auswertet und die Zuordnung zu der zutreffenden Musterzahnfarbe lernt,
- wobei, in einem Auswertschritt,
- eine Aufnahmevorrichtung, insbesondere eine Kamera eines Endgeräts, wie eines Smartphones, oder ein Scanner vorgesehen ist, mit welcher ein Bild eines Hilfskörpers mit vorbekannter, ggf. virtuell erzeugten, Musterzahnfarbe zusammen mit mindestens einem Zahn aufgenommen wird,
- die Aufnahmevorrichtung mindestens zwei Bilder der Kombination des zu bestimmenden Zahns und des Hilfskörpers aus unterschiedlichen Aufnahmewinkeln erfasst und der Auswertvorrichtung zuleitet, und
- die Auswertvorrichtung, basierend auf der gelernten Zuordnung zu der zutreffenden, ggf. virtuell erzeugten, Musterzahnfarbe, die erfassten Bilder auswertet und die Zahnfarbe des zu bestimmenden Zahns gemäß einem Referenzwert, wie einem gängigen Zahnschlüssel, z.B. A1, B2 usw., ausgibt.

2. Verfahren nach Anspruch 1, **dadurch gekennzeichnet, dass**, in dem vorbereitenden Schritt, die Iteration des lernenden Algorithmus beendet wird, wenn die Genauigkeit der Zuordnung zu der zutreffenden Musterzahnfarbe einen Schwellwert übersteigt, oder die Änderung der in der Iteration bereitgestellten Daten gegenüber den Daten der vorigen Iteration einen Schwellwert unterschreitet

3. Verfahren nach Anspruch 1 oder 2, **dadurch gekennzeichnet, dass**, in Beendigung des vorbereitenden Schritts, die Auswertvorrichtung oder deren Daten in eine Cloud verbracht wird oder werden, und, in dem Auswertschritt, die Zuleitung der mindestens zwei Bilder zur Cloud erfolgt.

4. Verfahren nach einem der vorhergehenden Ansprüche, **dadurch gekennzeichnet, dass** das Endgerät die Zahnfarbe des zu bestimmenden Zahns ausgibt.

5. Verfahren nach einem der vorhergehenden Ansprüche, **dadurch gekennzeichnet, dass** der Hilfskörper Referenzpunkte aufweist und, in dem Auswertschritt, die Auswertung nur dann beginnt, wenn Referenzpunkte in dem auszuwertenden Bild erkannt werden, und insbesondere im Falle des Fehlens von Referenzpunkten ein anderes Bild anfordert.

6. Verfahren nach einem der vorhergehenden Ansprüche, **dadurch gekennzeichnet, dass**, in dem Auswertschritt, die Auswertvorrichtung das auszuwertende Bild, insbesondere nach Erkennen von Referenzpunkten, in Segmente aufteilt.

7. Verfahren nach Anspruch 6, **dadurch gekennzeichnet, dass**, in dem Auswertschritt, die Auswertvorrichtung ein Segment aus den Segmenten als Zahnsegment festlegt, wenn das Segment eine Fläche im Wesentlichen in Zahnform und mit zahnähnlich geringen Farb- und Helligkeitsunterschieden hat.

8. Verfahren nach Anspruch 6 oder 7, **dadurch gekennzeichnet, dass**, in dem Auswertschritt, die Auswertvorrichtung ein Segment aus den Segmenten als Hilfskörpersegment festlegt.

9. Verfahren nach Anspruch 6, 7 oder 8, **dadurch gekennzeichnet, dass**, in dem Auswertschritt, die Auswertvorrichtung in einem Segment, insbesondere dem Zahnsegment und/oder dem Hilfskörpersegment, nach Reflexionen sucht und die Auswertung nur dann fortsetzt, wenn nur Reflexionen unterhalb eines vorgegebenen Schwellwert erkannt werden, und insbesondere ein anderes Bild anfordert, wenn die Reflexionen den vorgegebenen Schwellwert übersteigen.

10. Verfahren nach einem der vorhergehenden Ansprüche, **dadurch gekennzeichnet, dass**, in dem Auswertschritt, die Auswertvorrichtung dominante Farben eines Segments, insbesondere des Zahnsegments, bestimmt und die Auswertung, basierend auf der gelernten Zuordnung, insbesondere also unter Berücksichtigung unterschiedlicher Lichtverhältnisse, nach einer Methode des geringsten Farbabstands vornimmt.

11. Verfahren nach einem der vorhergehenden Ansprüche, **dadurch gekennzeichnet, dass**, in dem Auswertschritt, die Auswertvorrichtung die Auswertung auch basierend auf einem Vergleich der Farb- und Helligkeitswerte des oder der Hilfskörpersegmente und des Zahnsegments vornimmt.

12. Verfahren nach einem der vorhergehenden Ansprüche, **dadurch gekennzeichnet, dass** die Aufnahmevorrichtung wie die Kamera, oder das Endgerät, einen Umgebungslichtsensor aufweist, dessen Ausgangssignal der Auswertvorrichtung zugeleitet wird.

13. Verfahren nach einem der vorhergehenden Ansprüche, **dadurch gekennzeichnet, dass** die Auswertvorrichtung, nach einer vorgegebenen Anzahl von Auswertungen, den vorbereitenden Schritt unter Einbeziehung der erfolgten Auswertungen erneut vornimmt.

14. Verfahren nach einem der vorhergehenden Ansprüche, **dadurch gekennzeichnet, dass** die Auswertvorrichtung eine Geometrieerfassungseinheit aufweist, die basierend auf einer Ausrichtung oder Nicht-Parallelität von Segmentgrenzen ein Warnsignal abgibt, und dass die Auswertvorrichtung das Warnsignal als Hinweis auf eine erwünschte zu ändernde Ausrichtung des Endgeräts auf dessen Bildschirm darstellt.

15. Verfahren nach einem der vorhergehenden Ansprüche, **dadurch gekennzeichnet, dass** bekannte, in der Praxis auftretende Zahnformen zur Festlegung der Segmente und/oder zur Verbesserung der Geometrieerfassung in der Auswertvorrichtung abgespeichert sind und mit den erfassten Formen bzw. Segmentgrenzen verglichen werden.

16. Verfahren nach einem der vorhergehenden Ansprüche, **dadurch gekennzeichnet, dass** die Aufnahmevorrichtung als Kamera ausgebildet ist und die Auswertvorrichtung eine App eines Smartphones umfasst, welche App mindestens einen Teil der Auswertung durch die Auswertvorrichtung leistet.

17. Verfahren nach einem der vorhergehenden Ansprüche, **dadurch gekennzeichnet, dass** die Aufnahmevorrichtung als Scanner ausgebildet ist, in welchen Scanner ein Computer, insbesondere ein Minicomputer wie ein Raspberry Pi, integriert wird, der mindestens einen Teil der Auswertung durch die Auswertvorrichtung leistet.

18. Verfahren zur Festlegung einer Zahnfarbe, wobei
- eine Auswertvorrichtung einen iterativen lernenden Algorithmus aufweist, der, in einem vorbereitenden Schritt, basierend auf mindestens einer vorbekannten, RGB-Zahnfarbe, Bilder bei unterschiedlichen Lichtverhältnissen und Aufnahmewinkeln erfasst und auswertet und die Zuordnung zu der zutreffenden RGB-Zahnfarbe lernt,
- wobei, in einem Auswertschritt,
- eine Aufnahmevorrichtung, insbesondere eine Kamera eines Endgeräts, wie eines Smartphones, oder ein Scanner vorgesehen ist, mit welcher ein Bild eines Hilfskörpers mit vorbekannter RGB-Zahnfarbe zusammen mit mindestens einem Zahn aufgenommen wird,
- die Aufnahmevorrichtung mindestens zwei Bilder der Kombination des zu bestimmenden Zahns und des Hilfskörpers aus unterschiedlichen Aufnahmewinkeln erfasst und der Auswertvorrichtung zuleitet, und
- die Auswertvorrichtung, basierend auf der gelernten Zuordnung zu der zutreffendenRGB-Zahnfarbe, die erfassten Bilder auswertet und die Zahnfarbe des zu bestimmenden Zahns gemäß einem Referenzwert, wie einem gängigen Zahnschlüssel, z.B. A1, B2 usw., ausgibt.
